# DEMANDE DE BREVET EUROPEEN

(11) **EP 2 036 536 A1**
(43) Date de publication de la demande: **18.03.2009**
(21) Numéro de dépôt: 08164227.4
(22) Date de dépôt: 12.09.2008
(51) Int. Cl.: A61K 8/64, A61K 8/36, A61K 8/81, A61K 8/84, A61Q 5/06, A61K 8/88

(54) **Procédé capillaire comprenant l'application d'une polyamine et d'un acide gras**

(30) Priorité: 14.09.2007 FR 0757563
(71) Demandeur: L'Oréal, 75008 Paris (FR)
(72) Inventeur: Dorkel, Jocelyne, 95130, Le Plessis - Bouchard (FR); Vic, Gabin, 45400, Semoy (FR); Livoreil, Aude, 75006, Paris (FR)
(74) Mandataire: Bourdeau, Françoise

(57) **Abrégé**

L'invention a pour objet un procédé cosmétique capillaire non colorant, comprenant l'application d'une composition comprenant, dans un milieu cosmétiquement acceptable, au moins une polyamine, au moins un acide gras insaturé ou un de ses sels organique ou minéral.

## Description

L'invention a pour objet un procédé cosmétique capillaire non colorant, comprenant l'application sur les cheveux d'une composition comprenant, dans un milieu cosmétiquement acceptable, au moins une polyamine, au moins un acide gras insaturé ou un de ses sels organique ou minéral. Elle vise également l'utilisation de cette composition pour le soin des cheveux, notamment pour le soin des cheveux frisés, notamment pour une application antifrisottis.

### Problème posé :

Il existe des produits coiffants qui facilitent le brushing des cheveux frisottés ou ondulés. Ces produits contiennent généralement un corps gras, comme les alcools gras dans le cas des crèmes lissantes ou une silicone (Hydra-Liss®, Oléo-Relax®). Ces produits sont performants juste après application mais leur effet disparaît après shampooing. Il y a donc un besoin pour des compositions qui apportent une aide au brushing, une maîtrise de la chevelure et du volume et dont les effets sont résistants aux shampooings, tout en apportant aux cheveux de bonnes propriétés cosmétiques comme de la douceur, du lissage ou de la facilité de démêlage améliorée.

### Solution apportée :

La Demanderesse a mis en évidence que des compositions non colorantes contenant une polyamine et un acide gras insaturé facilitent le brushing et apportent une maîtrise du flou et du volume de la chevelure qui résiste à plusieurs shampooings. Dans le cas de cheveux frisés, on observe une maîtrise de la boucle et parfois une détente de la boucle.

L'invention a pour objet un procédé cosmétique capillaire non colorant, comprenant l'application d'une composition comprenant, dans un milieu cosmétiquement acceptable, au moins une polyamine, au moins un acide gras insaturé ou un de ses sels organique ou minéral, la polyamine étant une polylysine ou une polyéthylène imine.

Encore un autre objet de l'invention concerne l'utilisation de ces compositions cosmétiques non colorante pour le soin des cheveux, notamment pour le soin des cheveux frisés, notamment pour une application antifrisottis.

Par « *procédé non colorant* », on entend au sens de la présente invention, un procédé qui n'est pas destiné à conférer aux cheveux une couleur artificielle avec utilisation de colorants capillaires directs ou d'oxydation, à des concentrations efficaces.

### Description des éléments de l'invention

### 1- Les polyamines

Par polyamines, nous comprenons :
- les polyethyleneimines (PEI) comme notamment :
   le LUPASOL G35, FG, WF, SK, PS, HF, PO100, BO150 et P de BASF les PEI éthoxylées ou à tronçons polyethers : par exemple le Lupasol SC61B, SC62J ou HEO1 de BASF et les composés décrits dans le brevet WO9720879 et WO9723456, et ceux décrits dans l'article H Petersen et al, Macromolecules, 2002, 35, p6867 ;
   les PEI-polysaccharides, les PEI-mono, di et trisaccharide comme la PEI-Glucose, PEI-Galactose, PEI-Maltose, PEI-cellobiose, PEI-maltotriose,
   les PEI-Polyvinylalcool
   les PEI à chaîne grasse carbonée comme le LUPASOL ESA 51685 et LU157 de BASF,
   les PEI-PDMS comme le Mackamer PAVS de Mclntyre.
- en particulier, la poly-epsilon-lysine, notamment de Chisso et ses dérivés, notamment les dérivés siliconés XPS2, XPS4, XPS13 et les polylysine-PEG telles que celles décrites dans le brevet WO 00/071601 et dans l'article GL Kenausis et al, Journal of Physical Chemistry B, 2000, 104, p3298

La concentration en polyamine(s) utilisée dans les compositions selon la présente invention est comprise entre 0,01 et 50%, de préférence entre 0,05 et 30%, plus préférentiellement entre 0,1 et 20% en poids par rapport au poids total de la composition.

### 2- Les acides gras insaturés non siliconés naturels ou synthétique

Les acides gras utilisés dans la présente invention comportent au moins un groupement acide carboxylique et une chaîne alkyle linéaire ou ramifiée, insaturée contenant une ou plusieurs doubles ou triples liaisons carbone-carbone, ayant de 6 à 30 atomes de carbone et de préférence 12 à 22 atomes de carbone.

Ces acides peuvent être sous forme de sel minéral (sodium, potassium, calcium) ou organique (ammonium, monoéthanolamine)

On peut citer, les acides gras préférés suivants :

Acide crotonique, acide sorbique, acide décylénique, acide caproléique, acide undécylénique, acide lauroléique, acide myristoléique, acide pentadécénoïque, acide palmitoléique, acide palmitélaïdique, acide oléique, acide élaïdique, acide vaccénique, acide linoléique, acide gamma-linolénique, acide alpha-linolénique, acide stéradonique, acide parinarique ou stéaridonique, acide gadoléique, acide dihomo-linoléique, acide dihomo-gamma-linolénique, acide arachidonique, acide timnodonique ou éicosapentaènoïque, acide érucique, acide brassidique, acide cétoléique, acide adrénique, acide clupanodonique, acide docosahexaènoïque, acide nervonique ou sélacholéïque, acide eicosadiénoïque, acide eicosatriénoïque, acide docosadiénoïque, acide docosatétraénoïque, acide docosapentaénoïque.

Les acides gras particulièrement préférés sont l'acide oléique, l'acide linoléique et l'acide linolénique et leurs mélanges.

On préfère tout particulièrement l'acide oléique.

Ces acides gras peuvent se présenter sous forme d'huile ou sous forme de cire.

Une huile, au sens de la présente invention, est un composé lipophile, liquide à température ambiante (environ 25 °C), à changement d'état solide/liquide réversible.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope.

La concentration en acide(s) gras utilisée dans les compositions selon la présente invention est comprise entre 0,01 et 30%, de préférence entre 0,1 et 15% en poids par rapport au poids total de la composition.

Avantageusement, selon l'invention, l'acide gras insaturé est l'acide oléique.

Les compositions sont encore améliorées par l'ajout d'une silicone. Cette silicone apporte du glissant à l'application, un très bon mouillage de la fibre et améliore les propriétés de démêlage sur cheveux humides.

### 3- Les silicones

Les silicones utilisables dans les compositions selon la présente invention peuvent être linéaires, cyclique, ramifiées ou non ramifiées, volatiles ou non volatiles. Elles peuvent se présenter sous forme d'huiles, de résines ou de gommes, elles peuvent en particulier être des polyorganosiloxanes insolubles dans le milieu cosmétiquement acceptable.

Les organopolysiloxanes sont définis plus en détail dans l'ouvrage de Walter NOLL "Chemistry and Technology of Silicones" (1968) Academic Press. Ils peuvent être volatils ou non volatils.

Lorsqu'elles sont volatiles, les silicones sont plus particulièrement choisies parmi celles possédant un point d'ébullition compris entre 60° C et 260° C, et plus particulièrement encore parmi :
(i) les silicones volatiles cycliques comportant de 3 à 7 atomes de silicium et, de préférence, 4 à 5. Il s'agit, par exemple, de l'octaméthylcyclotétrasiloxane commercialisé notamment sous le nom de "VOLATILE
   SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 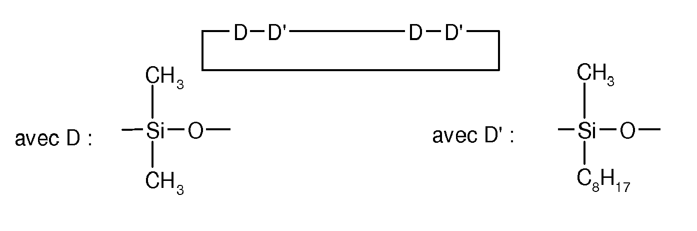 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant notamment une viscosité inférieure ou égale à 5.10-6m2/s à 25 °C mesurée ----. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan.76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicone, les polyorganosiloxanes modifiés par des groupements organofonctionnels ainsi que leurs mélanges.
Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C6-C24 tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C12)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C1-C4 ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FRA-85 16334.
- des groupements acyloxyalkyle tels que, par exemple, les polyorganosiloxanes décrits dans le brevet US-A-4957732.
- des groupements anioniques du type acide carboxylique comme,par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2- hydroxyalkylsulfonate ; 2-hydroxyalkylthiosulfate tels que les produits commercialisés par la société GOLDSCHMIDT sous les dénominations "ABIL® S201" et "ABIL® S255".
- des groupements hydroxyacylamino, comme les polyorganosiloxanes décrits dans la demande EP 342 834. On peut citer, par exemple, le produit Q2-8413 de la société DOW CORNING.

Les huiles de silicone utilisables dans les compositions selon l'invention sont des polyméthylsiloxanes volatiles ou non, à chaîne siliconée linéaire ou cyclique, liquides ou pâteuse à température ambiante, notamment les cyclopolydiméthylsiloxanes (cyclométhicones) telles que la cyclopentasiloxane, cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl siloxysilicates, les polyméthyl-phénylsiloxanes ; et leurs mélanges.

Les gommes de silicone utilisables dans les compositions selon l'invention sont des polydiorganosiloxanes de masse moléculaire élevée, comprise entre 200 000 et 2 000 000, utilisées seules ou en mélange dans un solvant choisi parmi les silicones volatiles, les huiles polydiméthylsiloxanes, les huiles polyméthylphénylsiloxanes, les huiles polydiphényldiméthylsiloxanes, les isoparaffines, le chlorure de méthylène, le pentane, les hydrocarbures ou leurs mélanges.

On utilise de préférence une gomme de silicone de poids moléculaire inférieur à 1 500 000. Les gommes de silicone sont par exemple des gommes polydiméthylsiloxanes, des gommes polyphénylméthylsiloxanes, des gommes poly(diphénylsiloxane diméthylsiloxanes), des gommes poly(diméthylsiloxaneméthylvinylsiloxanes), des poly(diméthylsiloxanephénylméthylsiloxanes), des gommes poly(diphénylsiloxane diméthylsiloxaneméthylvinylsiloxanes).

Ces gommes de silicones peuvent être terminées en bout de chaîne par des groupements triméthylsilyls ou diméthylhydroxysilyls ou hydroxylés.

Les résines de silicone utilisables dans les compositions selon l'invention sont des systèmes siloxaniques réticulés renfermant les unités R2SiO2/2 RSiO3/2 SiO4 /2, dans lesquelles R représente un groupe hydrocarbonés possédant de 1 à 6 atomes de carbone ou un groupe phényle. Parmi ces produits, ceux particulièrement préférés sont ceux dans lesquelles R désigne un radical alkyl inférieur (C1-C6) ou un radical phényle.

Les composés siliconés préférés sont les silicones volatiles, c'est-à-dire dont la pression de vapeur à 20°C sont supérieures à 0,1 mm de Hg, de préférence comprise entre 0,1 et 300 mm Hg, plus préférentiellement encore entre 0,5 et 200 mm Hg.

Les silicones cycliques volatiles comme la cyclopentadiméthylsiloxane commercialisée sous le nom de DC 245 fluid par Dow Corning, la cyclohexadiméthylsiloxane commercialisée sous le nom de DC 246 fluid par Dow Corning et leur mélange commercialisée sous le nom de DC 345 fluid par Dow Corning

Les mélanges de gommes de dimethiconol et de silicones volatiles, en particulier le mélange polydiméthylsiloxane alpha-oméga dihydroxylé et de cyclopentadimethylsiloxane (14/85,3) commercialisé sous le nom de DC 1501 fluid par Dow corning.

La concentration en silicone(s) utilisée dans les compositions selon la présente invention est comprise entre 0,1 et 30%, de préférence entre 0,5 et 20%, plus préférentiellement entre 1 et 10% en poids par rapport au poids total de la composition.

La composition contenant la polyamine, le corps gras et la silicone est spécialement efficace sous sa forme biphasique. Des compositions émulsionnées en sérum ou en crème peuvent être obtenues par ajout de tensioactifs ou d'épaississants appropriés et présentent les mêmes propriétés « d'antifrisottis durable ».

Les compositions selon l'invention, avec ou sans silicone, peuvent contenir un ou plusieurs agents tensioactifs.

Le tensio-actif utile dans l'émulsion de l'invention peut être n'importe quel tensio-actif connu de la technique. Ce tensio-actif peut être anionique, amphothère, cationique ou non ionique.

A titre de tensio-actifs utiles dans l'invention, on peut citer les tensioactifs anioniques non siliconés notamment les sels des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Les sels de ces composés sont par exemple des sels alcalins, notamment de sodium, sels d'ammonium, sels d'amines, sels d'aminoalcools ou sels de magnésium. Parmi les tensioactifs anioniques on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 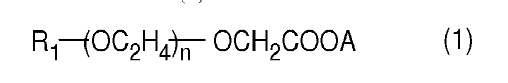 dans laquelle :
R1 représente un groupement alkyle ou alkylaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
A représente un atome d'hydrogène, un ammonium, ion sodium, potassium, lithium ou magnésium ou un reste monoéthanolamine ou triéthanolamine. On peut également utiliser des mélanges de composés de formule (1) en particulier des mélanges dans lesquels les groupements R1 sont différents.

Parmi tous ces tensioactifs anioniques, on préfère utiliser les sels d'alkylsulfates et d'alkyléthersulfates, ainsi que leurs mélanges.

A titre de tensio-actifs amphotères non siliconés, on peut citer des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant par exemple carboxylate, sulfonate, sulfate, phosphate ou phosphonate ; on peut citer encore les alkyl (C8-C20) bétaïnes, les sulfobétaïnes, les alkyl (C8-C20) amidoalkyl (C1-C6) bétaïnes ou les alkyl (C8-C20) amidoalkyl (C1-C6) sulfobétaïnes.

Parmi les dérivés d'amines, on peut citer les produits commercialisés sous les dénomination MIRANOL, tels que décrits dans les brevets US-2 528 378 et US-2 781 354 et de structures :

R2 -CONHCH2CH2 -N(R3)(R4)(CH2COO-) (2)

dans laquelle : R2 désigne un radical alkyle dérivé d'un acide R2-COOH présent dans l'huile de coprah hydrolysée, un radical heptyle, nonyle ou undécyle, R3 désigne un groupement bêta-hydroxyéthyle et R4 un groupement carboxyméthyle ; et

R5-CONHCH2CH2-N(B)(C) (3)

dans laquelle
B représente -CH2CH2OX', C représente -(CH2)z -Y', avec z = 1 ou 2,
X' désigne le groupement -CH2CH2-COOH ou un atome d'hydrogène
Y' désigne -COOH ou le radical -CH2 - CHOH - SO3H
R5 désigne un radical alkyle d'un acide R9 -COOH présent dans l'huile de coprah ou dans l'huile de lin hydrolysée, un radical alkyle, notamment en C7, C9, C11 ou C13, un radical alkyle en C17 et sa forme iso, un radical C17 insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL C2M concentré par la société RHONE POULENC.

A titre de tensioactif(s) non ionique(s) non siliconé(s), on peut citer les tensioactifs non-ioniques bien connus en soi (voir notamment à cet égard "Handbook of Surfactants" par M.R. PORTER, éditions Blackie & Son (Glasgow and London), 1991, pp 116-178). Ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10 - C14) amines ou les oxydes de N-acylaminopropylmorpholine. Selon un mode de réalisation de l'invention, les alkylpolyglycosides constituent des tensio-actifs non-ioniques utiles dans le cadre de la présente invention.

A titre de tensioactifs cationiques non siliconé, on peut utiliser les tensioactifs bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer,
- ceux qui présentent la formule suivante : 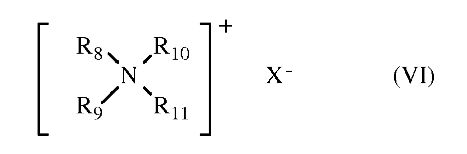 dans laquelle les radicaux R8 à R11, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C2-C6), alkylamide, alkyl(C12-C22)amidoalkyle(C2-C6), alkyl(C12-C22)acétate, hydroxyalkyle, comportant environ de 1 à 30 atomes de carbone ; X est un anion choisi dans le groupe des halogénures, phosphates, acétates, lactates, alkyl(C2-C6)sulfates, alkyl- ou alkylaryl-sulfonates ;
- les sels d'ammonium quaternaire de l'imidazoline, comme par exemple ceux de formule suivante : 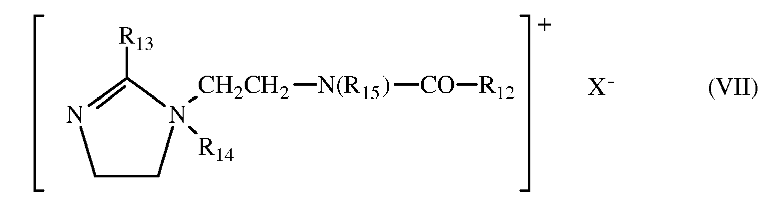 dans laquelle R12 représente un radical alcényle ou alkyle comportant de 8 à 30 atomes de carbone, par exemple dérivés des acides gras du suif, R13 représente un atome d'hydrogène, un radical alkyle en C1-C4 ou un radical alcényle ou alkyle comportant de 8 à 30 atomes de carbone, R14 représente un radical alkyle en C1-C4, R15 représente un atome d'hydrogène, un radical alkyle en C1-C4, X- est un anion choisi dans le groupe des halogénures, phosphates, acétates, lactates, alkylsulfates, alkyl- ou alkylaryl-sulfonates. De préférence, R12 et R13 désignent un mélange de radicaux alcényle ou alkyle comportant de 12 à 21 atomes de carbone, par exemple dérivés des acides gras du suif, R14 désigne un radical méthyle, R15 désigne un atome d'hydrogène. Un tel produit est par exemple commercialisé sous la dénomination REWOQUAT® W 75 par la société REWO ;
- les sels de diammonium quaternaire de formule (VIII) : 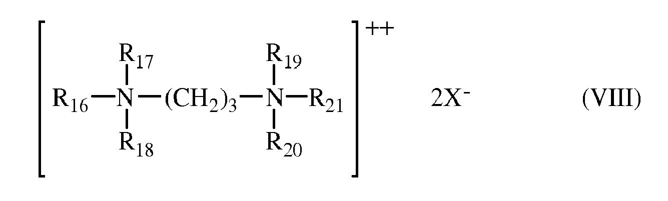 dans laquelle R16 désigne un radical aliphatique comportant environ de 16 à 30 atomes de carbone, R17, R18, R19, R20 et R21 , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IX) suivante : 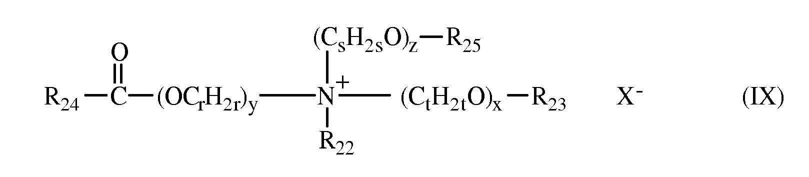 dans laquelle :
   R22 est choisi parmi les radicaux alkyles en C1-C6 et les radicaux hydroxyalkyles ou dihydroxyalkyles en C1-C6 ;
   R23 est choisi parmi :
   - le radical 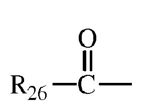
   - les radicaux R27 hydrocarbonés en C1-C22, linéaires ou ramifiés, saturés ou insaturés,
   - l'atome d'hydrogène,
   R25 est choisi parmi :
   - le radical 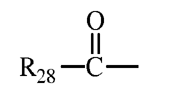
   - les radicaux R29 hydrocarbonés en C1-C6, linéaires ou ramifiés, saturés ou insaturés,
   - l'atome d'hydrogène,
   R24, R26 et R28, identiques ou différents, sont choisis parmi les radicaux hydrocarbonés en C7-C21, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X- est un anion simple ou complexe, organique ou inorganique ;
   sous réserve que la somme x + y + z vaut de 1 à 15, que lorsque x vaut 0 alors R23 désigne R27 et que lorsque z vaut 0 alors R25 désigne R29.

Les radicaux alkyles R22 peuvent être linéaires ou ramifiés et plus particulièrement linéaires.

De préférence R22 désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R23 est un radical R27 hydrocarboné, il peut être long et avoir de 12 à 22 atomes de carbone, ou court et avoir de 1 à 3 atomes de carbone.

Lorsque R25 est un radical R29 hydrocarboné, il a de préférence 1 à 3 atomes de carbone.

Avantageusement, R24, R26 et R28, identiques ou différents, sont choisis parmi les radicaux hydrocarbonés en C11-C21, linéaires ou ramifiés, saturés ou insaturés, et plus particulièrement parmi les radicaux alkyle et alcényle en C11-C21, linéaires ou ramifiés, saturés ou insaturés.
De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate. On utilise plus particulièrement dans la composition selon l'invention, les sels d'ammonium de formule (IX) dans laquelle :
- R22 désigne un radical méthyle ou éthyle,
- x et y sont égaux à 1 ;
- z est égal à 0 ou 1 ;
- r, s et t sont égaux à 2 ;
- R23 est choisi parmi :
   - le radical 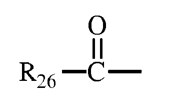
   - les radicaux méthyle, éthyle ou hydrocarbonés en C14-C22,
   - l'atome d'hydrogène ;
- R25 est choisi parmi :
   - le radical 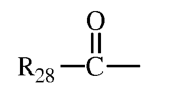
   - l'atome d'hydrogène ;
- R24, R26 et R28, identiques ou différents, sont choisis parmi les radicaux hydrocarbonés en C13-C17, linéaires ou ramifiés, saturés ou insaturés, et de préférence parmi les radicaux alkyles et alcényles en C13-C17, linéaires ou ramifiés, saturés ou insaturés.

Avantageusement, les radicaux hydrocarbonés sont linéaires.
On peut citer par exemple les composés de formule (IX) tels que les sels (chlorure ou méthylsulfate notamment) de diacyloxyéthyl-diméthylammonium, de diacyloxyéthylhydroxyéthylméthylammonium, de monoacyloxyéthyl-dihydroxyéthylméthylammonium, de triacyloxyéthylméthylammonium, de monoacyloxyéthylhydroxyéthyldiméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

A titre de tensioactifs cationique, on préfère le mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthylméthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (VI), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.

Les tensioactifs cationiques particulièrement préférés sont choisis parmi les sels d'ammonium quaternaire, et en particulier parmi le chlorure de béhényltriméthylammonium et le chlorure de palmitylamidopropyltriméthylammonium.

Les tensio-actifs non siliconés sont généralement présents dans l'émulsion de la présente invention dans des proportions variant de 0,1% à 30% en poids de matière active ; plus préférentiellement entre 0,5% et 15% en poids de composition.
- A titre de tensioactifs utilisables dans la présente invention on peut aussi particulièrement citer les tensioactifs siliconés décrits notamment dans le brevet FR2818902. Les tensioactifs siliconés utilisables dans la présente invention sont ceux bien connus de l'homme du métier. Ils peuvent être hydrosolubles, spontanément hydrodispersibles ou non hydrosolubles. De préférence, ils sont hydrosolubles ou spontanément hydrodispersibles.
   Les tensioactifs siliconés sont, par exemple, choisis parmi les composés de formules générales suivantes : 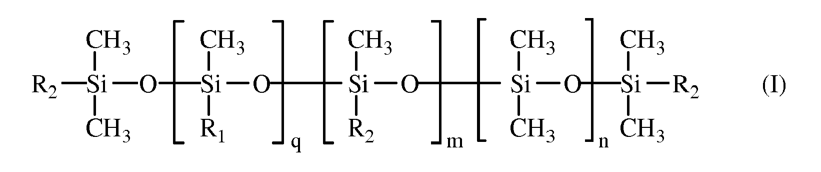 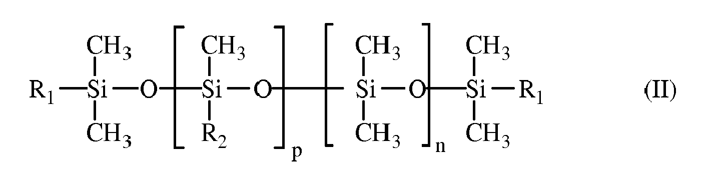 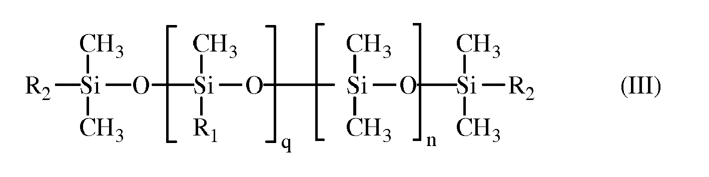 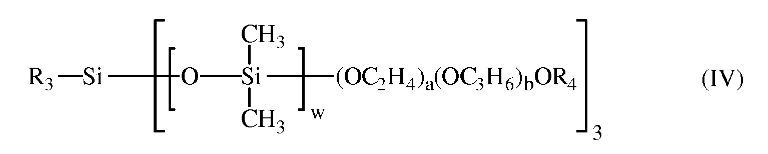 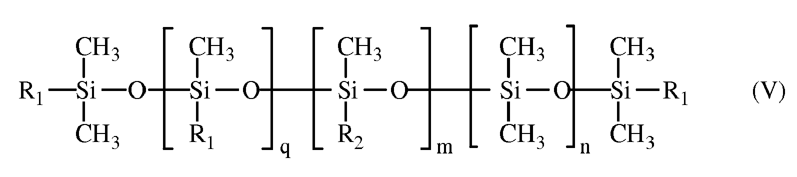 formules dans lesquelles :
   - R1, identique ou différent, représente un groupe alkyle, linéaire ou ramifié, en C1-C30, ou phényle ;
   - R2, identique ou différent, représente -CcH2c-O-(C2H4O)a-(C3H6O)b-R5 ou
   - CcH2c-O-(C4H8O)a-R5 ;
   - R3 et R4, identiques ou différents, désignent chacun un groupe alkyle, linéaire ou ramifié, en C1-C12, et de préférence un groupe méthyle ;
   - R5, identique ou différent, est choisi parmi un atome d'hydrogène, un groupe alkyle, linéaire ou ramifié, comportant de 1 à 12 atomes de carbone, un groupe alcoxy, linéaire ou ramifié, comportant de 1 à 6 atomes de carbone, un groupe acyle, linéaire ou ramifié, comportant de 2 à 12 atomes de carbone, un groupe hydroxyle, - SO3M, -OCOR6, aminoalcoxy en C1-C6 éventuellement substitué sur l'amine, aminoacyle en C2-C6 éventuellement substitué sur l'amine, -NHCH2CH2COOM,
   - N(CH2CH2COOM)2, aminoalkyle en C1-C12 éventuellement substitué sur l'amine et sur la chaîne alkyle, carboxyacyle en C1-C30, un groupement phosphono éventuellement substitué par un ou deux groupes aminoalkyle en C1-C12 substitués,
   - CO(CH2)dCOOM, -OCOCHR7(CH2)dCOOM, -NHCO(CH2)dOH, -NH3Y ;
   - M, identique ou différent, désigne un atome d'hydrogène, Na, K, Li, NH4 ou une amine organique ;
   - R6 désigne un groupe alkyle, linéaire ou ramifié, en C1-C30,
   - R7 désigne un atome d'hydrogène ou un groupe SO3M ;
   - d varie de 1 à 10 ;
   - m varie de 0 à 20 ;
   - n varie de 0 à 500 ;
   - p varie de 1 à 50 ;
   - q varie de 0 à 20 ;
   - a varie de 0 à 50 ;
   - b varie de 0 à 50 ;
   - a + b est supérieur ou égal à 1 ;
   - c varie de 0 à 4 ;
   - w varie de 1 à 100 ;
   - Y représente un anion minéral ou organique monovalent tel qu'un halogénure (chlorure, bromure), un sulfate, ou un carboxylate (acétate, lactate, citrate).

De préférence, on utilise des tensioactifs siliconés répondant aux formules générales (IV) ou (VII) telles que définies ci-dessus, et plus particulièrement, ceux répondant aux formules (IV) ou (VII) dans lesquelles au moins l'une des, et de préférence toutes les conditions suivantes sont satisfaites :
- c est égal à 2 ou 3 ;
- R1 désigne le groupe méthyle ;
- R5 représente un atome d'hydrogène, un groupe méthyle ou un groupe acétyle et de préférence un atome d'hydrogène ;
- a varie de 1 à 25 et plus particulièrement de 2 à 25 ;
- b varie de 0 à 25, de préférence de 10 à 20 ;
- n varie de 0 à 100 ;
- p varie de 1 à 20.

Les tensioactifs siliconés les plus particulièrement préférés sont, par exemple, ceux vendus sous les dénominations commerciales FLUID DC 193 et DC 5225C par la société DOW CORNING, SILWET® L 77 par la société OSI et MAZIL® 756 par la société MAZER PPG, le mélange LAURYL PEG/PPG-18/18 METHICONE (and) POLOXAMER 407 (and) DODECENE commercialisé par Dow Corning sous le nom de DC 5200

Les tensioactifs siliconés les plus particulièrement préférés sont, par exemple, ceux vendus sous les dénominations commerciales FLUID DC 193 et DC 5225C par la société DOW CORNING, SILWET® L 77 par la société OSI et MAZIL® 756 par la société MAZER PPG, le mélange LAURYL PEG/PPG-18/18 METHICONE (and) POLOXAMER 407 (and) DODECENE commercialisé par Dow Corning sous le nom de DC 5200
Bien que tout type de ionicité puisse être utilisé pour les émulsions de la présente invention, les tensio-actifs présentant une HLB (hydrophilic Lipophilic Balance) inférieure à 10 sont préférés. En particulier, les tensioactifs non ioniques utilisés dans les compositions de l'invention présentent de préférence une HLB allant de 1,5 à 10, et mieux encore de 1,5 à 7. La HLB ou balance hydrophile-lipophile du ou des tensioactifs non ioniques utilisés selon l'invention est la HLB selon GRIFFIN définie dans la publication J. Soc. Cosm. Chem. 1954 (Volume 5), pages 249-256.

Le ou les tensioactifs sont contenus en général en une quantité allant de 0,01 à 30 % en poids, de préférence de 0,1 à 30% en poids, et mieux encore de 0,2 à 15 % en poids par rapport au poids total de la composition.

Selon un mode de réalisation préféré, le ou les tensio-actifs présents dans l'émulsion sont choisis parmi les tensio-actifs siliconés.

Les tensio-actifs siliconés sont préférentiellement présents dans l'émulsion de la présente invention dans des proportions variant de 0,1% à 30% en poids d'émulsion; préférentiellement entre 0,2% et 15% en poids d'émulsion.

Avantageusement, dans le procédé selon l'invention, la composition comprend une silicone polyoxyalkylénée et un tensioactif.

Avantageusement, dans le procédé selon l'invention, la composition comprend une huile non siliconée.

Avantageusement, dans les compositions non colorantes de l'invention, l'acide gras insaturé est l'acide oléique.

Plus avantageusement, elle se présente sous la forme d'émulsion E/H.

Plus avantageusement encore, elle comprend au moins un tensioactif siliconé.

### Procédés selon l'invention

Plusieurs procédés de mise en oeuvre sont envisageables :

### P1 : Le traitement de la fibre peut être un procédé en un temps.

Sur des cheveux secs ou shampooinés, on applique la composition selon l'invention. On peut laisser pauser à température ambiante ou sous chaleur (casque) pour une durée de 30s à 30 min. On peut éventuellement rincer. On réalise, éventuellement, ensuite un lissage avec une pince plate ou un brushing avec un sèche-cheveux.

P2 : Un procédé spécialement intéressant consiste à combiner le traitement selon l'invention avec des traitements de la fibre existant. De cette façon on prépare la fibre pour rendre l'efficacité plus importante (par décapage de la fibre et/ou par mordançage de la fibre)

Ainsi dans une première étape on applique un réducteur de permanente ou une permanente ou une coloration d'oxydation ou une décoloration ou un shampooing ou un produit de coiffage ou un défrisage alcalin ou un soin et dans une seconde étape on procède à un des procédés décrits plus haut.

P3 : Un autre procédé spécialement intéressant consiste à appliquer le traitement selon l'invention avant des traitements de la fibre existants, connus pour être spécialement dégradant. De cette façon on protège la fibre lors du traitement. On peut ensuite éventuellement démaquiller la fibre.

Ainsi après avoir appliqué la composition de l'invention selon un des procédés déjà décrits on applique un réducteur de permanente ou une permanente ou une coloration d'oxydation ou une décoloration ou un défrisage alcalin.

P4 : Un autre procédé particulièrement intéressant consiste à appliquer la composition dans un solvant cosmétique selon les procédés déjà décrits juste après une coloration d'oxydation ou directe ou un mélange de ces colorants afin de protéger l'éclat de la couleur aux shampooings.

La composition selon l'invention peut contenir en outre au moins un adjuvant choisi parmi les polymères non-ioniques, anioniques, cationiques et amphotères différents de ceux de l'invention, les céramides et pseudo-céramides, les vitamines et pro-vitamines dont le panthénol, les filtres solaires hydrosolubles et liposolubles, liquides ou solides, siliconés ou non siliconés, les composés solides tels que les pigments, les agents nacrants ou opacifiants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les glycols, les agents de pénétration, les parfums et les agents conservateurs.

L'homme de métier veillera à choisir les éventuels additifs et leur quantité de manière à ce qu'ils ne nuisent pas aux propriétés des compositions selon la présente invention.

Ces additifs sont présents dans la composition selon l'invention en une quantité allant de 0 à 20 % en poids par rapport au poids total de la composition.

Les compositions selon l'invention peuvent se présenter sous toutes les formes appropriées pour une application topique, notamment sous forme de solutions du type lotion ou sérum, éventuellement biphasiques ; sous forme de gels aqueux ; sous forme d'émulsions obtenues par dispersion d'une phase grasse dans une phase aqueuse (H/E) ou inversement (E/H), de consistance liquide plus ou moins épaisse telle que des laits et des crèmes plus ou moins onctueuses ; sous forme de mousse, sous forme de spray, ou d'aérosol ; ou encore sous forme de brosse ou de stick.

De préférence, les compositions de l'invention sont des émulsions E/H. Dans ces émulsions E/H, la phase huileuse est de préférence une silicone volatile. La phase huileuse peut contenir, à la place de la silicone volatile ou en addition à cette silicone volatile, une autre huile.

Ces émulsions E/H contiennent, à titre de tensioactif préféré, un ou plusieurs tensioactifs siliconés.

Lorsque la composition selon l'invention est conditionnée dans un dispositif aérosol, elle comprend au moins un agent propulseur, qui peut être choisi parmi les hydrocarbures volatiles, tels que le N-butane, le propane, l'isobutane, le pentane, les hydrocarbures halogénés et leurs mélanges. On peut également utiliser en tant que propulseur le gaz carbonique, le protoxyde d'azote, le diméthyléther (DME), l'azote, ou l'air comprimé. On peut aussi utiliser des mélanges de propulseurs. De préférence, on utilise le diméthyl éther.

Avantageusement, l'agent propulseur est présent à une concentration comprise entre 5 et 90% en poids par rapport au poids total de la composition dans le dispositif aérosol, et, plus particulièrement, à une concentration comprise entre 10 et 60%.

De préférence, la composition selon l'invention est non colorante.

La composition selon l'invention peut notamment être utilisée en application non rincée sur les cheveux.

Les exemples suivants illustrent la présente invention.

### Exemples

Sur une chevelure de cheveux bouclés préalablement shampooinés on applique 5 à 10g des compositions suivantes. Les cheveux sont séchés en effectuant un brushing au sèche-cheveux. Les cheveux sont lissés, le volume est diminué et les frisottis sont contrôlés. Après un shampooing l'effet est toujours présent.
Les pourcentages sont exprimés en poids par rapport au poids total de la composition.

### Exemples 1 :

### Solution A

| | |
|---|---|
| | |
| POLYLYSINE EN SOLUTION A 25% DANS L'EAU. | 8 |
| ACIDE OLEIQUE | 0,4 |
| EAU DESIONISEE | 91,6 |
| Total: | 100 |

### Solution B

| | |
|---|---|
| | |
| MELANGE POLY DIMETHYLSILOXANE | |
| ALPHA-OMEGA DIHYDROXYLE/ CYCLOPENTA DIMETHYLSILOXANE (14.7/85.3)⁽¹⁾ | 100 |

On prépare une formule contenant 80% de la solution A et 20% de la solution B pour application sur cheveux. On applique le mélange sur des cheveux bouclés. Les cheveux sont traités et on ne constate pas la présence de frisottis.

### Exemple 2

### Solution A

| | |
|---|---|
| | |
| POLYETHYLENE IMINE (Poids moléculaire: 750000) EN SOLUTION AQUEUSE A 50%⁽²⁾ | 10 |
| ACIDE OLEIQUE | 2 |
| EAU DESIONISEE | 88 |
| Total | 100 |

### Solution B

| | |
|---|---|
| | |
| MELANGE POLY DIMETHYLSILOXANE ALPHA-OMEGA DIHYDROXYLE / CYCLOPENTA DIMETHYLSILOXANE (14.7/85.3)⁽¹⁾ | 100 |

On prépare une formule contenant 80% de la solution A er 20% de la solution B pour application sur cheveux. On applique le mélange sur des cheveux bouclés. Les cheveux sont lisses et doux et on ne constate pas la présence de frisottis.

### Exemple 3 :

| | |
|---|---|
| | |
| POLYLYSINE EN SOLUTION A 25% DANS L'EAU. | 8 |
| ACIDE OLEIQUE | 0,4 |
| MELANGE POLY DIMETHYLSILOXANE ALPHA-OMEGA DIHYDROXYLE / CYCLOPENTA DIMETHYLSILOXANE (14.7/85.3)⁽¹⁾ | 8 |
| MELANGE DE POLY DIMETHYLSILOXANE OXYETHYLENE OXYPROPYLENE (15 OE/18 OP), DE CYCLOPENTA DIMETHYLSILOXANE ET D'EAU (10/88/2) | 8 |
| CYCLOPENTA DIMETHYLSILOXANE commercialisé par Dow Corning sous la dénomination DOW CORNING 245 Fluid® | 11 |
| EAU DESIONISEE | 64,6 |
| Total: | 100 |

On applique directement la composition ci-dessus sur cheveux. Les cheveux sont lisses et doux et les frisottis supprimés.

### Exemple 4

| | |
|---|---|
| | |
| POLYETHYLENE IMINE (PM: 750000) EN SOLUTION AQUEUSE A 50% | 10 |
| ACIDE OLEIQUE | 2 |
| CYCLOPENTA DIMETHYLSILOXANE | 8 |

| | |
|---|---|
| | |
| MELANGE DE POLY DIMETHYLSILOXANE OXYETHYLENE OXYPROPYLENE (18 OE/18 OP), DE CYCLOPENTA DIMETHYLSILOXANE ET D'EAU (10/88/2) (Dow Corning 5225C Formulation Acid®) | 8 |
| MELANGE DE POLY DIMETHYLSILOXANE ALPHA-OMEGA DIHYDROXYLE / CYCLOPENTA DIMETHYLSILOXANE(14.7/85.3)⁽¹⁾ | 11 |
| EAU DESIONISEE | 61 |
| Total: | 100 |

On applique directement la composition ci-dessus sur cheveux, avec un séchage au brushing ou un sèche-cheveux. Les cheveux sont lisses et doux et les frisottis supprimés.

### Exemple 5 :

| | Concentration |
|---|---|
| POLYLYSINE EN SOLUTION A 25% DANS L'EAU. | 8.0 |
| ACIDE OLEIQUE | 0.4 |
| MELANGE POLY DIMETHYLSILOXANE ALPHA-OMEGA DIHYDROXYLE / CYCLOPENTA DIMETHYLSILOXANE (14.7/85.3) ⁽¹⁾ | 8.0 |
| CYCLOPENTA DIMETHYLSILOXANE (Dow Corning 245 Fluid®) | 11.0 |
| POLYACRYLAMIDE (and) C13-14 ISOPARAFFIN (and) LAURETH-7 (SEPIGEL 305 commercialisé par la Société SEPPIC) | 1.0 |
| EAU DESIONISEE | 71.6 |
| TOTAL | 100 |

On applique directement la composition ci-dessus sur cheveux, avec un séchage au brushing ou un sèche-cheveux. Les cheveux sont lisses et doux et les frisottis supprimés.

### Exemple 6

| | Concentration |
|---|---|
| POLYLYSINE EN SOLUTION A 25% DANS L'EAU. | 8.0 |
| ACIDE OLEIQUE | 8.0 |
| MELANGE POLY DIMETHYLSILOXANE ALPHA-OMEGA DIHYDROXYLE / CYCLOPENTA DIMETHYLSILOXANE (14.7/85.3) ⁽¹⁾ | 8.0 |
| CYCLOPENTA DIMETHYLSILOXANE (Dow Corning 245 Fluid) | 11.0 |
| EAU DESIONISEE | 65 |
| TOTAL | 100 |

On applique directement la composition ci-dessus sur cheveux, avec un frisage au sèche-cheveux. Les cheveux sont lisses et doux et les frisottis supprimés.
(1) MELANGE POLY DIMETHYLSILOXANE ALPHA-OMEGA DIHYDROXYLE / CYCLOPENTA DIMETHYLSILOXANE (14.7/85.3) commercialisée par Dow Corning sous la dénomination DOW CORNING 1501 Fluid®
(2) POLYETHYLENE IMINE (PM: 750000) EN SOLUTION AQUEUSE A 50% commercialisée par BASF sous la dénomination LUPASOL P®

## Revendications

1. Procédé cosmétique capillaire non colorant, comprenant l'application d'une composition comprenant, dans un milieu cosmétiquement acceptable, au moins une polyamine, au moins un acide gras insaturé ou un de ses sels organique ou minéral, **caractérisé par le fait que** la polyamine est une polylysine ou une polyéthylène imine.

2. Procédé selon l'une quelconque des revendications précédentes, **caractérisé par le fait que** la concentration en polyamine(s) est comprise entre 0,01 et 50%, de préférence entre 0,05 et 30%, plus préférentiellement entre 0,1 et 20% en poids par rapport au poids total de la composition.

3. Procédé selon l'une quelconque des revendications précédentes, **caractérisé par le fait que** l'acide gras est choisi parmi les acides suivants : acide crotonique, acide sorbique, acide décylénique, acide caproléique, acide undécylénique, acide lauroléique, acide myristoléique, acide pentadécénoïque, acide palmitoléique, acide palmitélaïdique, acide oléique, acide élaïdique, acide vaccénique, acide linoléique, acide gamma-linolénique, acide alpha-linolénique, acide stéradonique, acide parinarique ou stéaridonique, acide gadoléique, acide dihomo-linoléique, acide dihomo-gamma-linolénique, acide arachidonique, acide timnodonique ou éicosapentaènoïque, acide érucique, acide brassidique, acide cétoléique, acide adrénique, acide clupanodonique, acide docosahexaènoïque, acide nervonique ou sélacholéïque, acide eicosadiénoïque, acide eicosatriénoïque, acide docosadiénoïque, acide docosatétraénoïque, acide docosapentaénoïque.

4. Procédé selon l'une quelconque des revendications précédentes, **caractérisé par le fait que** la concentration en acide(s) gras est comprise entre 0,01 et 30%, de préférence entre 0,1 et 15% en poids par rapport au poids total de la composition.

5. Procédé selon l'une quelconque des revendications précédentes, **caractérisé par le fait que** la composition comprend en outre une silicone.

6. Procédé selon la revendication 5, **caractérisé par le fait que** la concentration en silicone(s) est comprise entre 0,1 et 30%, de préférence entre 0,5 et 20%, plus préférentiellement entre 1 et 10% en poids par rapport au poids total de la composition.

7. Procédé selon l'une quelconque des revendications précédentes, **caractérisé par le fait que** la composition comprend au ou plusieurs agents tensioactifs choisis parmi les tensio-actifs anionique, amphotère, cationique ou non ionique.

8. Procédé selon la revendication 7, **caractérisé par le fait que** le ou les tensioactifs sont contenus en une quantité allant de 0,01 à 30 % en poids, de préférence de 0,1 à 30% en poids, et mieux encore de 0,2 à 15 % en poids par rapport au poids total de la composition.

9. Procédé selon l'une quelconque des revendications précédentes, **caractérisé par le fait que** la composition comprend, en outre, au moins un adjuvant choisi parmi les polymères non-ioniques, anioniques, cationiques et amphotères différents de ceux de l'invention, les céramides et pseudo-céramides, les vitamines et pro-vitamines dont le panthénol, les filtres solaires hydrosolubles et liposolubles, liquides ou solides, siliconés ou non siliconés, les composés solides tels que les pigments, les agents nacrants ou opacifiants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les glycols, les agents de pénétration, les parfums et les agents conservateurs.

10. Procédé selon l'une quelconque des revendications précédentes, **caractérisé par le fait que** la composition se présente sous la forme d'émulsion, obtenue par dispersion d'une phase aqueuse dans une phase huileuse (E/H).

11. Procédé selon l'une quelconque des revendications précédentes, **caractérisé par le fait qu'**on réalise un lissage avec une pince plate ou un brushing avec un sèche-cheveux, après avoir appliqué la composition selon l'une quelconque des revendications précédentes.

12. Procédé selon l'une quelconque des revendications précédentes, **caractérisé par le fait qu'**on prépare la fibre par décapage de la fibre et/ou par mordançage.

13. Procédé selon l'une quelconque des revendications précédentes, **caractérisé par le fait qu'**on applique au préalable, un réducteur de permanente ou une permanente ou une coloration d'oxydation ou une décoloration ou un shampooing ou un produit de coiffage ou un défrisage alcalin ou un soin.

14. Utilisation d'une composition comprenant, dans un milieu cosmétiquement acceptable, au moins une polyamine, au moins un acide gras insaturé ou un de ses sels organique ou minéral, pour le soin des cheveux, notamment pour le soin des cheveux frisés.

15. Utilisation selon la revendication 14 pour une application antifrisottis.
